Europäisches Patentamt

(19) European Patent Office

Office européen des brevets

(11) Publication number: **0 233 249 B1**

# (12) EUROPEAN PATENT SPECIFICATION

(45) Date of publication of patent specification: **24.11.93**  (51) Int. Cl.⁵: **A61K 49/04**, C07C 233/67

(21) Application number: **86905063.3**

(22) Date of filing: **01.08.86**

(86) International application number:
**PCT/US86/01590**

(87) International publication number:
**WO 87/00757 (12.02.87 87/04)**

(54) **NON-IONIC POLYOL CONTRAST MEDIA FROM IONIC CONTRAST MEDIA.**

(30) Priority: **09.08.85 US 764274**

(43) Date of publication of application:
**26.08.87 Bulletin 87/35**

(45) Publication of the grant of the patent:
**24.11.93 Bulletin 93/47**

(84) Designated Contracting States:
**AT BE CH DE FR GB IT LI LU NL SE**

(73) Proprietor: **COOK IMAGING CORPORATION**
**3333 North Torrey Pines Court**
**La Jolla California 92037(US)**

(72) Inventor: **SOVAK, Milos**
**Post Office Box 2494**
**Rancho Santa Fe, CA 92067(US)**
Inventor: **RANGANATHAN, Ramachandran**
**6043 Stresemann**
**San Diego, CA 92122(US)**

(74) Representative: **Harrison, David Christopher**
**et al**
**MEWBURN ELLIS**
**2 Cursitor Street**
**London EC4A 1BO (GB)**

Rank Xerox (UK) Business Services
(3.10/3.6/3.3.1)

**Description**

This invention is related to contrast media used in the field of medical imaging with X-rays. It is particularly related to non-ionic contrast media.

Medical imaging with X-rays depends to a great extent upon radiographic contrast media (CM). An ideal CM, designed to mix with the body fluids, should be economically feasible, chemically stable, highly water soluble, readily injectable, and biologically inert. CM of the prior art, generally based on salts of derivatized triiodinated benzene moieties, meet the first four criteria, but they induce adverse clinical effects. Such toxicity extends from their ionicity, solution hyperosmolality vis-a-vis the body fluids and chemotoxicity, reflecting their relatively high hydrophobicity.

Non-ionic, less hyperosmolal, less hydrophobic but more costly compounds exist, of which Iohexol/Iopamidol/Metrizamide are used clincially in the United States. Metrizamide suffers from hydrolytic instability and thus must be dispensed in lyophilized form and reconstituted prior to use. Solutions of some other non-ionic, stable CM have, however, higher osmolality and can thus elicit pain when injected into the arteries. Other compounds are, at elevated concentrations, not persistently water soluble. All current non-ionic CM, while less toxic than the prior art, are much more costly.

Consequently, despite the large number of compounds which have been prepared, there is substantial interest in producing a non-ionic CM improved both pharmacologically and economically. To this end, it is essential once the ring has been iodinated that subsequent steps are few and have high yields. Furthermore, the iodinated substrate, as well as the reactants that are employed for additional functionalization, should be inexpensive.

There is extensive patent literature concerned with non-ionic contrast media and their method of preparation. See particularly, U.S. Patents Nos. 4,364,921; 4,341,756; 4,250,113; 4,021,481; 4,001,323, 3,702,866; 3,701,771; and 3,622,616. See also, "Radiocontrast Agents", Volume 73 of the Handbook of Experimental Pharmacology, Springer, New York, 1985, which provides a comprehensive review of the field as of the time of publication.

The compounds of claim 1 and 3 are useful as non-ionic contrast media. The compound of claim 2 is an intermediate.

The media may be prepared by selective and efficient polyhydroxylalkylation of the nitrogen of an acylamido substituted triiodobenzioc acid with a polyhydroxyalkyl halide in an aqueous medium under weakly basic conditions. The polyhydroxylalkylated acylamidobenzioc acid is then activated for formation of functionalized benzamides. Alternatively, N-polyhydroxylalkylation can be carried out on fully functionalized benzamide intermediates made from ionic or non-ionic precursors. The methodologies provide a novel and efficient synthetic approach to both known and novel non-ionic contrast media. The combination of low viscosity and low osmolality in the products has not previously been obtained in a clinically useful contrast medium.

The starting materials may be 5-acylamido substituted triiodobenzoic acids, where the 3-position will be substituted with a substituted amino group or a carboxamido group. The starting materials will normally have at least about 10 carbon atoms, and usually from 0 to 2, more usually from 0 to 1, hydroxyl group. The product will usually have less than 20 carbon atoms, more usually fewer than about 18 carbon atoms, and will have at least three nitrogen atoms, of which at least one will be substituted to an annular carbon atom while one or both of the nitrogen atoms may be amido. Acyl groups bound to nitrogen will generally have from 1 to 4 carbon atoms, usually from 2 to 3 carbon atoms, and from 0 to 3 oxy substituents, more usually from 0 to 2 oxy substituents. Alkyl substituents will be generally of from 1 to 3 carbon atoms, more usually of from 1 to 2 carbon atoms, and having from 0 to 3 hydroxyl groups, more usually from 0 to 2 hydroxyl groups.

The following flowchart indicates one synthetic strategy. TIB intends triiodobenzene, where the vertical line indicates the groups associated with the horizontal lines are bound at the 1, 3, and 5 positions, respectively. The numbers over the arrows indicate the reaction, with the legend indicating the reagents and conditions for the reaction.

$$TIB \begin{cases} CO_2H \\ Y \\ NHCOR \end{cases} \quad \overset{(1)}{\longrightarrow} \quad TIB \begin{cases} CO_2H \\ Y^1 \\ N(R^5)COR \end{cases}$$

$$\longrightarrow (2)$$

$$TIB \begin{cases} COX \\ Y^2 \\ N(R^6)COR^7 \end{cases} \quad \overset{(3)}{\longleftarrow} \quad TIB \begin{cases} CO_2H \\ Y^2 \\ N(R^6)COR^7 \end{cases}$$

$$\longrightarrow (4)$$

$$TIB \begin{cases} CONR^8R^9 \\ Y^2 \\ N(R^6)COR^7 \end{cases} \quad \overset{(5)}{\longrightarrow} \quad TIB \begin{cases} CONR^{8'}R^{9'} \\ Y^4 \\ N(R^5)COR \end{cases}$$

(1)   halohydrin of 2 to 5 carbon atoms and 1 to 4 oxy groups; aqueous base, pH 9-13, 60-100°C, 0.5-6hr.

(2)   AcZ, Z = chloro or AcO, where Ac is an acyl group of from 2 to 3 carbon atoms; tert.-amine, 50-80°C, 1-6hr.

(3)   G-Cl (G-inorganic or organic acyl group); 50-80°C; 0.25-3hr.

(4)   $HNR^8R^9$; tert.-amine, 35-75°C.

(5)   (a) $OH^-$; (b) neutralization, optionally acidification when acetonides are present.

The symbols are defined as follows:

TIB = 2,4,6-triiodobenzene;

Y = $NR^1R^2$ or $CONR^3R^4$ or $CH_2NR^2COR^3$

R = an aliphatic group of from 1 to 3, usually 2 to 3 carbon atoms, having from 0 to 2, usually 0 to 1 oxy groups;

$R^1$ = hydrogen, an aliphatic group of from 1 to 3, usually 1 to 2 carbon atoms, having from 0 to 2, usually 0 to 1 oxy groups, an aliphatic acyl group of from 1 to 3, usually 1 to 2 carbon atoms, and from 0 to 2, usually 0 to 1 oxy groups;

$R^2$ = the same or different from $R^1$, usually $R^2$ will be hydrogen or an aliphatic group; at least one of $R^1$

and $R^2$ being other than hydrogen;

$R^3$ = hydrogen or an aliphatic group of from 1 to 3, usually 1 to 2 carbon atoms, having from 0 to 2, usually 0 to 1, oxy groups or acyloxy groups;

$R^4$ = the same or different from $R^3$, usually hydrogen;

$R^5$ = mono- or polyoxyalkyl from 2 to 5, usually 2 to 4, preferably 3 to 4 carbon toms, having from 1 to 4, usually 1 to 2 oxy groups;

$Y^1$ = $NR^1 R^{2\prime}$ or $CONR^3 R^4$;

$R^{2\prime}$ = the same as $R^2$ with the proviso that when $R^2$ is hydrogen and $R^1$ is acyl or an aliphatic group, then $R^{2\prime}$ includes mono- or polyoxyalkyl of from 2 to 5, usually 2 to 4, preferably 3 to 4 carbon atoms having from 1 to 3, usually 1 to 2 oxy groups;

$R^6$ = the same as $R^5$, except all hydroxyl groups of $R^5$ are acyloxy groups, where Ac is bonded to the hydroxyl oxygen;

$R^7$ = the same as R, except all hydroxyl groups of $R^5$ are acyloxy groups, where Ac is bonded to the hydroxyl oxygen;

$Y^2$ = the same as $Y^1$, except all hydroxyl groups of $Y^1$ are acyloxy groups, where Ac is bonded to the hydroxyl oxygen;

$R^8$ and $R^9$ = the same or different and are hydrogen or alkyl of from 1 to 4 carbon atoms having 0 to 3, usually 1 to 3 oxy groups, the total number of carbon atoms being not greater than about 6, usually not greater than about 4;

$R^{8\prime}$ and $R^{9\prime}$ = the same as $R^8$ and $R^9$ except they exclude alkoxy groups as substituents;

Ac = an aliphatic acyl group of 2 to 3 carbon atoms, particularly acetyl;

X = halo or 2-oxypyridyl, N-oxysuccinimidyl or iso-ureido;

$Y^4$ = $Y^1$ or Y.

Each of the stages will now be considered in detail. In these methods starting compound will be an acylamido triiodo substituted benzoic acid, where the other substituent is a carboxamido group or an acylamido group. Desirably, the starting materials may be ionic contrast media or their iodinated precursors, readily commercially available and inexpensive. Such compounds include derivatives or triiodo-3,5-diaminobenzoic acid, diatrizoate, 3,5-diacetamido-2,4,6-triiodobenzoic acid, and metrizoate, the N-mono-methyl derivative of diatrizoate, and derivatives of 5-aminoisophthalic acid, iothalamate, 5-acetamido-2,4,6-triiodo-N-methyl-isophthalamic acid; and ioxithalamic acid, 5-acetamido-2,4,6-triiodo-N-(2-hydroxyethyl)-isophthalamic acid or its immediate precursor, the corresponding N-(2-acetoxyethyl) compound. While generally available ionic contrast media are preferred as starting materials, any of the triiodobenzoic acid derivatives substituted at the 3 and 5 positions with amino and carboxy groups having various useful substituents may be employed.

The method will now be described in further detail. The first step is the reaction of the acylamido substituted triiodobenzioc acid with a halohydrin of from 2 to 5 carbon atoms, usually 3 to 4 carbon atoms, particularly a chlorohydrin, preferably where the chloro group is a primary or secondary chloro group, there being from 1 to 4 oxy groups, at least one of the oxy groups being hydroxy to provide a vicinal halohydrin. The reaction will be carried out in aqueous base, normally a basic solution of at least pH 9, generally from about pH 9 to pH 14, more usually from about pH 9.5 to pH 13.5. Stoichiometric amounts of the halohydrin may be employed, usually a small excess, not exceeding two molar excess, usually not exceeding one molar excess. The pH is maintained during the course of the reaction. Temperatures will normally be at least about 45°C and not exceeding about 100°C, preferably between 45°C to 95°C. The reaction is carried out until completion, which can be monitored by TLC or HPLC. Generally, less than 2hr is required, frequently less than 1hr. An aqueous medium is employed which may or may not have cosolvents. Since an aqueous medium suffices, cosolvents will usually not be employed.

At completion of the reaction, the product need not be isolated and purified, rather, the medium may be neutralized to a mildly acidic pH, usually from about pH 4 to pH 6 and the solvents removed, e.g., azeotroped with an appropriate cosolvent, e.g., pyridine or toluene. The residue may then be used directly in the next step.

The next stage is the protection stage, where hydroxyl groups will be reacted with an appropriate reagent which is stable under the reaction conditions of the next successive steps. Since the next successive steps will involve acidic reagents, the protective groups will be those which will be able to survive the subsequent reactions. The reagents employed for the protection will of course be reactive so as to react with the hydroxyl groups and any available amino group, will not interfere with the reactions of the carboxyl group to form an amide, and wild allow for easy recovery of the product free of the protective groups. Furthermore, since economics are important to the synthetic strategy, normally inexpensive groups will be employed. However, other groups could be used less efficiently and less economically.

Of particular interest is the use of acylhalides and acyl anhydrides of from 1 to 3, preferably 2 carbon atoms, particularly acetic anhydride. With acetic anhydride, the anhydride may serve as the solvent and will therefore be in substantial excess, the particular amount will usually be at least about 2- to 3-fold molar excess. With other agents, the agents themselves may either be used as the solvent, when appropriate, or an inert solvent may be employed such as acetonitrile, ethyl acetate or dichloromethane. In addition to the anhydride, an activating catalyst will be employed, particularly a tertiary amino compound, more particularly pyridine. The temperatures will be higher than room temperature, generally in the range of about 40-60°C, and the reaction will usually require about 1-6hr, depending upon the particular reagent and the size of the reaction batch. The course of the reaction may be followed by thin-layer chromatography (TLC).

Workup will normally involve removal of the solvents by evaporation and azeotroping, as appropriate. The residue may then be dissolved in water and the aqueous layer extracted with a water immiscible polar organic solvent, e.g., an ester, conveniently ethyl acetate, in admixture with a nonpolar solvent, such as toluene. The aqueous layer may then be acidified to precipitate the hydroxy-protected benzoic acid and the precipitate dissolved into an organic extractant, conveniently the same organic extractant, and the organic extracts combined. The product can then be isolated in conventional ways.

The hydroxy-protected benzoic acid compound is then activated, so as to be reactive with an aliphatic amine. A variety of ways are available for activation of the carboxy groups. O-Acylureas can be formed, by employing carbodiimides or the like. Active esters may be prepared, such as N-oxysuccinimide, 2-acyloxypyridyl, nitrophenyl, chlorophenyl, or the like. While the particular manner in which the carboxyl group is activated is not critical to this invention, the preferred method is to prepare the acyl chloride employing an inorganic or organic acid halide, particularly an inorganic halide such as thionyl chloride, sulfuryl chloride, phosphorus pentachloride, or the like. Of particular interest is the use of thionyl chloride, where the thionyl chloride may be used as the solvent and be present in excess, usually at least about 1 to 4 molar excess, and the reactant dissolved in the thionyl chloride. Alternatively, the compound may be dissolved in an inert solvent such as dichloromethane or ethyl acetate and thionyl chloride employed in a small excess, usually 2 to 4 molar excess. The mixture will be heated at an elevated temperature, generally from about 50-75°C for a sufficient time for the reaction to go to completion, generally from about 0.25 to 3hr. The reaction may be monitored by TLC. The thionyl chloride and other incipient solvents may then be removed by evaporation and appropriate azeotroping of the reside to remove any residual thionyl chloride, and the resulting product dissolved in an inert polar organic solvent, e.g., an ester, followed by washing with bicarbonate and drying of the organic layer.

The activated carboxyl, particularly the acyl halide, may then be combined in an inert organic polar solvent, conveniently an ether or an amide, more conveniently dioxane or dimethylacetamide, with an acid-neutralizing compound, conveniently a tertiary amino compound, or in a mixture of an inert organic polar solvent, preferably acetone or dichloromethane (which gives two phases) with water, in the presence of an inorganic base, preferably a carbonate or bicarbonate such as $Na_2CO_3$, $K_2CO_3$, or $NaHCO_3$. The amino compound may be ammonia or alkylamino of from 1 to 4 carbon atoms, having from 0 to 3, usually from 0 to 2 hydroxy groups, which may be protected or unprotected, when protected, as ethers, particularly acetals or ketals, more particularly acetonide. The reaction is carried out under mild conditions at room temperature or at an elevated temperature, generally from about 40-70°C until completion, which will usually require about 0.5hr and less than 12hr, usually less than 9hr.

The workup follows generally the same procedure as prior workups, in that the solvents are evaporated, the product dissolved in an appropriate polar organic solvent and washed with water with or without added sodium chloride. The organic layers may then be dried and the solvent removed by evaporation. In each instance, the isolation steps are conventional.

The hydroxyl groups are then deprotected employing a basic medium, usually basic alkanolic medium, particularly methanol, the pH being at least about 10 and hydroxyl concentration being less than 1 normal. The reaction may be carried out under mild conditions, usually ambient temperatures being satisfactory, the reaction usually being complete in less than about 2hr. Volatile materials may then be removed by evaporation and the residue neutralized with aqueous acid, also under ambient conditions. Conveniently, a pH of 1 to 2 may be employed to remove acetonide functions when they are present. Desirably, the product may be further purified by desalting with an appropriate ion exchange resin.

A wide variety of compound may be made in accordance with the subject invention. Of particular interest are the novel compounds 5-(2,3-dihydroxypropylacetamido)-2,4,6-triiodo-N-(2,3-dihydroxypropyl-)N'-(2-hydroxyethyl)-isophthalamide (compound VIII in the Experimental Section) and 5-(N-2,3-dihydroxypropylacetamido)-2,4,6,-triiodo-N-methyl-N'-(1,3,4-trihydroxy-threo-but-2-yl)-isophthalamide (compound XVIII in the Experimental Section).

5

These particular compounds are found to have excellent properties as to toxicity, water solubility, osmolality, stability, viscosity and the like, factors predominantly important in angio- and urography.

It is also possible to produce novel non-ionic polyol contrast media beginning with non-iodinated compounds. For example, a 5-amino-N-(mono or poly)hydroxyalkyl-N'-(mono or poly)hydroxyalkyl-isophthalamide can be reacted with an iodine source, such as $KICl_2$ in an aqueous acid solution with heating. In this and the other compounds of this sequence, the substituents present on the benzene ring are preferably the same substituents indicated above to be preferred. The product of this first reaction is a 5-amino-2,4,6-triiodo-N-(mono or poly)hydroxyalkyl-5'-(mono or poly)hydroxyalkyl-isophthalamide.

This first intermediate is then reacted with an acylating compound, preferably an acylhalide or acylanhydride, most preferably an acylhalide such as acetyl chloride, to give the 5-acylamino derivative. This derivative can also be prepared from an ionic iodinated compound (e.g., ioxithalamic acid), by protecting the hydroxyls by acetylation, by activating the carboxyl, especially with an acid halide, and by reacting with an appropriate hydroxyalkylamine, particularly 1-amino-2,3-propanediol. See Examples 3 and 25 for details of the appropriate reactions. This derivative is then reacted with an epihalohydrin as described previously. A preferred method is to dissolve the derivative in 1,2-propanediol containing sodium bicarbonate and epichlorohydrin. This reaction is typically completed in approximately 1hr at 90°C.

A preferred starting material for use in this aspect of the invention is 5-amino-N-(2-hydroxyethyl)-N'-(2,3-dihydroxypropyl)isophthalamide, which is available commercially. Carrying out the reactions described above with $KICl_2$ acetylchloride, and epichlorohydrin gives 5-(N-2,3-dihydroxypropylacetamido)-2,4,6-triiodo-N-(2,3-dihydroxypropyl)-N'-(2-hydroxyethyl)isophthalamide, compound VIII in the Experimental Section, which can also be prepared by the techniques previously described. However, this particular method produces the desired compound in only three steps from a commercially available source via 5-(N-acetamido)-2,4,6-triiodo-N-(2,3-dihydroxypropyl)-N'-(2-hydroxyethyl)isophthalamide as an intermediate.

In preparing the subject compounds of the invention, an improved process for alkylating acylamidobenzene compounds has been discovered. The prior art has typically indicated that alkylation of such compounds with an alkylating agent, such as an alkylchloride or an epichlorohydrin, has required the presence of a weak organic base, such as triethylamine, or a strong organic base, such as sodium methoxide. It has been discovered that high yields of alkylated products can be achieved by carrying out the reaction in the presence of sodium bicarbonate. Typically, the acylamidobenzene compound is dissolved in an alcohol, typically an alcohol containing 2 to 4 carbon atoms and 1 to 3 oxy groups, such as 1,2-propanediol, and reacted with the alkylating agent, such as epichlorohydrin, in the presence of sodium bicarbonate. The reaction is particularly suited to converting acetamido compounds into the corresponding N-(2,3-dihydroxypropyl) acetamido compound.

The sodium bicarbonate is typically present in excess to ensure complete scavenging of any acid generated in the alkylation reaction. Example 37 of the following examples sets forth a complete example of this reaction, including times, temperatures, and molar ratios.

The subject compounds may be used as contrast media for angiography, urography and opacification of body cavities.

These compounds are suitable as opacifying compounds in all fields of application of water-soluble non-ionic X-ray contrast media, especially for intravasal, subarachnoid and various local applications for which presently available non-ionic contrast media are employed.

The subject compounds can be formulated in accordance with conventional techniques, using pharmaceutically acceptable organic or inorganic carrier substances suitable for parenteral or enteral application for administration to a patient. Conventional pharmaceutically acceptable carriers include but are not limited to water, saline solution, alcohols, vegetable oils, polyethylene glycols, gelatin, lactose, amylose, magnesium stearate, paraffin oils, fatty acid mono- and diglycerides, pentaerythritol fatty acid esters, hydroxymethylcellulose, polyvinyl pyrrolidone, talc.

Other additives which are conventional in galenic pharmacy include stabilizers, such as sodium EDTAate, calcium disodium EDTAate, physiologically compatible buffers or sodium chloride.

For parenteral application, useful solutions include oily or aqueous solutions, as well as suspensions or emulsions.

For intravenous administration, the subject compounds will normally be used in an aqueous medium, where the concentration will be about 15 to 80 vol. percent, the active agent per unit dosage being about 1 to 80g, usually 20 to 70g.

Preferred concentration in aqueous media will generally be from about 50-400mg I/ml, preferably about 100-400mg I/ml, with dosages running from about 5 to 500ml.

The following examples are offered by way of illustration.

6

EXPERIMENTAL

Example 1. Alkylation of ioxithalamic acid
5-Acetamido-2,4,6-triiodo-N-(2-hydroxyethyl)-isophthalamic acid (I) into:
5-(N-2,3-Dihydroxypropylacetamido)-2,4,6,-triiodo-N-(2-hydroxyethyl)-isophthalamic acid (II)

To ioxithalamic acid (161g, 0.25 moles) was added 1N sodium hydroide (250ml) and the pH adjusted with 5N NaOH to 10.5-10.6 at 85-90°C. 3-Chloro-1,2-propanediol (30.41g, 0.275 moles) was added and the pH readjusted to 10.5-10.6 with 5N NaOH, followed by further additions at 1hr (2.76, 0.025 moles) and at 2hr (2.76g, 0.025 moles). The reaction was complete at 2.5hr by TLC.

Glacial acetic acid (5ml) was added to pH 5, solvents were evaporated and the residue azeotroped with toluene (150ml) to obtain 294g of a mixture which was used without product isolation in the next step.

Example 2. Acetylation of N-alkylated ioxithalamic acid
5(N-2,3-Dihydroxypropylacetamido)-2,4,6,-triiodo-N-(2-hydroxyethyl)-isophthalamic acid (II) into:
5-(N-2,3-Diacetoxypropylacetamido)-2,4,6-triiodo-N-(2-acetoxyethyl)-isophthalamic acid (III)

The crude mixture (290g) from step one, containing the title compound (250mMoles), was suspended in acetic anhydride (500ml) and pyridine (19.76g, 250mMoles) and mechanically stirred at 65°C. By TLC, the acetylation was complete after 3hr.

The acetic anhydride and acetic acid were evaporated, and the residue azeotroped with toluene (100ml x 2). The residue was dissolved in saturated aqueous sodium bicarbonate (500ml) and ethyl acetate (200ml). The layers were separated, and the bicarbonate layer re-extracted with ethyl acetate (200ml x 2). The aqueous layer was acidified with concentrated hydrochloric acid to pH 0-1 to obtain a white precipitate which was extracted with ethyl acetate (3x200ml). The organic extracts were combined and washed with brine (100ml), and dried over $MgSO_4$. Removal of the solvent gave 206g of the product (III) as a white foam (97% yield).

Example 3. Acyl-chloride formation of N-alkylated, acetylated ioxithalamic acid
5(N-2,3-Diacetoxypropylacetamido)-2,4,6-triiodo-N-(2-acetoxyethyl)-isophthalamic acid (III) into:
5-(N-2,3-Diacetoxypropylacetamido)-2,4,6-triiodo-N-(2-acetoxyethyl)-isophthalamic acid chloride (IV)

The title compound (III) (250g, 243mMoles) was dissolved in thionyl chloride (400ml), and the reaction mixture heated at 60-65°C for 1hr to completion (by TLC). The thionyl chloride was evaporated on a rotary evaporator, the residue azeotroped with ethyl acetate (250ml x 2), the product dissolved in ethyl acetate (400ml), extracted with aqueous saturated bicarbonate (150ml x 2) and dried over $MgSO_4$ to give 202g of an off-white foam (96% yield).

Example 4. Amidation of alkylated, acetylated ioxithalamic acid chloride with trans-dioxepane (protected amino-threitol)
5-(N-2,3-Diacetoxypropylacetamido)-2,4,6-triiodo-N-(2-acetoxyethyl)-isophthalamic acid chloride (IV) into:
5-(N-2,3-Diacetoxypropylacetamido)-2,4,6-triiodo-N-2-acetoxyethyl)-N'-(trans-2,2-dimethyl-6-hydroxy-1,3,-dioxepan-5-yl)-isophthalamide (V)

The title compound (86.25g, 100mMoles) was dissolved in dimethylacetamide (200ml) to which was added triethylamine (13.9ml, 100mMoles) and trans-5-amino-2,2-dimethyl-6-hydroxy-1,3-dioxepane (19.3g, 120mMoles). The reaction mixture was stirred at room temperature for 8hr to completion (by TLC). The solvent was evaporated in vacuo and the residue dissolved in ethyl acetate (200ml). The solution was washed with water (3x50ml) and brine (2x50ml). Drying ($MgSO_4$) followed by solvent removal yielded the product (V) (96g) as an off-white foam (97% yield).

Example 5. Deprotection of alkylated acetylated ioxithalamic acid amidated with trans-dioxepane to aminothreitol derivative
5-(N-2,3-Diacetoxypropylacetamido)-2,4,6-triiodo-N-(2-acetoxyethyl)-N'-(trans-2,2-dimethyl-6-hydroxy-1,3-dioxepan-5-yl)-isophthalamide (V) into:
5-(N-2,3-Dihydroxypropylacetamido)-2,4,6-triiodo-N-(2-hydroxyethyl)-N'-(1,3,4-trihydroxy-threo-but-2-yl)-isophthalamide (VI)

7

The title compound (V) (4.94g, 5mMoles) was dissolved in methanol (20ml), the pH was adjusted to 12-13 with 5N sodium hydroxide, and the mixture was agitated for 1hr at 25°C to achieve complete deacetylation (by TLC). Upon evaporation to dryness, 15ml of 0.1N HCl was added (to pH 1-1.5), the solution stirred for 30min at 25°C to obtain the product (by HPLC) which, after evaporation of acid and redissolving in water, was desalted with AG-501 mixed bed ion exchange resin. The solution was decolorized with charcoal and the solvent removed in vacuo to obtain the product (VI) as a white powder (3.2g) (78% yield).

Example 6. Amidation of alkylated, acetylated ioxithalamic acid with 3-amino-1,2-propanediol
5-(N-2,3-Diacetoxypropylacetamido)-2,4,6-triiodo-N-(2-acetoxyethyl)-isophthalamic acid chloride (IV) into:
5-(N-2,3-Diacetoxypropylacetamido)-2,4,6-triiodo-N-(2-acetoxyethyl)-N'-(2,3-dihydroxypropy1)-isophthalamide (VII)

The title compound (IV) (86.25g, 100mMoles) was dissolved in dimethylacetamide (200ml) to which triethylamine (13.9g, 100mMoles) and 3-amino-1,2-propanediol (10.93g, 120mMoles) were added. The reaction was stirred at room temperature for 8hr to completion by TLC. The solvent was evaporated in vacuo and the product dissolved in tetrahydrofuran (75ml) and partitioned with water saturated with sodium chloride. The organic extract was washed with brine:1N hydrochloric acid (9:1, 50ml x 2), followed by brine water (1:1) (50ml x 2) and finally brine (40ml x 1). The organic layer was dried over $MgSO_4$ and the solvent was removed to give 80.6g of the product (VII) as an off-white foam (87.9% yield).

Example 7. Deprotection of alkylated, acetylated ioxithalamic acid amidated with 3-amino-1,2-propanediol
5-(N-2,3-Diacetoxypropylacetamido)-2,4,6-triiodo-N-(2-acetoxyethyl)-N'-(2,3-dihydroxypropyl)-isopthalamide (VII) into:
5-(N-2,3-Dihydroxypropylacetamido)-2,4,6-triiodo-N-(2,3-dihydroxypropyl)-N'-(2-hydroxyethyl)-isophthalamide (VIII)

The title compound (VII) (9.17g, 10mMoles) was dissolved in methanol (20ml), the pH adjusted to 13 with 5N sodium hydroxide and stirred at room temperature for 30min to achieve complete deacetylation (by TLC and HPLC). The solution was neutralized with Dowex 50 $H^+$ resin, and evaporated to give 7.8g of an off-white foam (99% yield). This product was dissolved in water and decolorized with charcoal. Removal of the solvent gave the product (VIII) as a white foam (6.3g) (80% yield).

NMR: ($^1H$, 80 MHz, DMSO-d6): 8.6 (2 H, broadened multiplet, carbamoyl N-H); 4.9-4.0 (5 H, broad singlet, exchangeable, hydroxyl protons); 4.1-2.8 (14 H, multiplet, protons on carbon bearing nitrogen and hydroxyl functions); 2.25 and 1.8 (3 H, pair of singlets, acetanilide methyl protons).

TLC: silica gel 70:30 $CHCl_3$:MeOH: rf (acetylated compound VII) 0.84; rf (product compound VIII) 0.20.

HPLC: aminopropyl Alltech, 10μ, 31ml/min of 87% acetonitrile/water.

rf: 6.1 and 7.5 for two isomers.

Elemental Analysis: Calculated for $C_{18}H_{24}I_3N_3O_8H_2O$: C,26.71; H,3.26; I,47.05; N,5.19%; Found: C,26.45, H,3.30; I,46.71; N,4.80%.

ALTERNATE SYNTHESIS OF COMPOUND (VIII)

Step 1. Alkylation or ioxithalamic acid
5-Acetamido-2,4,6-triiodo-N-(2-hydroxyethyl)-isophthalamic acid (I) into:
5-(N-2,3-dihydroxypropylacetamido)-2,4,6-triiodo-N-(2-hydroxyethyl)-isophthalamic acid (II)

To the ioxithalamic acid (161g, 0.25 moles) was added 1N sodium hydroxide (250ml), followed by calcium hydroxide (13.4g, 0.181 moles) and the suspension heated to 90°C. 1-Chloro-2,3-propanediol (37.3g, 0.338 moles) was added over 2 hours. The reaction was complete at 2.5hr by TLC.

Concentrated hydrochloric acid was added to pH 5.0, solvents were evaporated and the residue azeotroped with acetic acid (200ml) to obtain a mixture which was used without product isolation in the next step.

Step 2. Acetylation of N-alkylated ioxithalamic acid
5-(N-2,3-Dihydroxypropylacetamido)-2,4,6-triiodo-N-(2-hydroxyethyl)-isophthalamic acid (II) into:
5-(N-2,3-Diacetoxypropylacetamido)-2,4,6-triiodo-N-(2-acetoxyethyl)-isophthalamic acid (III)

The crude mixture from step one, containing the title compound (II) (185g, 0.25 moles), was mixed with pyridine (19.76g, 0.25 moles), and acetic anhydride (240ml, 2.54 moles) was added, maintaining the temperature at 70°C. By TLC, the acetylation was complete after 3hr.

The acetic anhydride and acetic acid were largely evaporated, and the residue was dissolved in water (250ml). The aqueous solution was washed with butyl acetate (50mlx3) and then was acidifed with concentrated hydrochloric acid to pH 0 to 1 to obtain a white precipitate which was extracted with dichloromethane (3x200ml). The organic extracts were combined, the solvent removed and replaced with 1,2-dichloroethane (350ml). Partial removal of the solvent gave a viscous solution that was dry enough for chlorination (containing the product in 93% yield).

Step 3. Acyl-Chloride formation of N-alkylated, acetylated ioxithalamic acid
5-(N-2,3-Diacetoxypropylacetamido)-2,4,6-triiodo-N-(2-acetoxyethyl)-isophthalamic acid (III) into:
5-(N-2,3-Diacetoxypropylacetamido)-2,4,6-triiodo-N-(2-aceteoxyethyl)-isophthalamic acid chloride (IV)

The title compound (III) (205g, 0.243 moles) in 1,2-dichloroethane (total volume 250ml) was mixed with thionyl chloride (53.2ml, 0.79 moles) at 70°C, and the reaction mixture maintained at 70°C for 2hrs to completion (by TLC). The solvents were evaporated on a rotary evaporator, and the residue azeotroped with 1,2-dichloroethane (100ml x 2). The product was dissolved in 1,2-dichloroethane (200ml), washed with aqueous saturated bicarbonate (150ml x 1) and the solvent removed to give a viscous solution (containing the product IV in 96% yield).

Step 4. Amidation of 3-(N-2-acetoxyethyl)-carbamoyl-5-(N-2,3-diacetoxypropyl)-acetyl-amino-2,4,6-trilodobenzoyl chloride (IV) with 3-aminopropane-1,2-diol into:
5-(N-2,3-Diacetoxypropylacetamido)-2,4,6-triiodo-N-(2-acetoxyethyl)-N'-(2,3-dihydroxypropyl)-isopthalamide (VII)

The title compound (IV) (240g, 0.27 moles) in 1,2-dichloroethane (total volume 160ml) was diluted with acetone (270ml) and added to a mixture of 3-amino-1,2-propanediol (30.4g, 0.334 moles), water (65ml), and sodium bicarbonate (23.4g, 0.278 moles). The mixture was heated at 55°C for 8hrs, when TLC indicated that the reaction had gone to completion. Water (500ml) was added and the solution was extracted with 1,2-dichloroethane containing 15% by volume of acetone (2 x 40ml). The aqueous layer was salted with sodium sulfate (140g) and was extracted with a mixture of dichlorormethane:n-propanol (9:1, 300ml). The dichloromethane was removed at atmospheric pressure, n-propanol (300ml) was added, and the solution was concentrated to a volume of 250ml. This solution was treated with Dowex-50-H+ resin to remove the excess 3-amino-1,2-propanediol, and the solution was charcoaled overnight under reflux. The charcoal was removed and the filtrate was freed of the solvent to obtain an off-white foam (VII) (220g).

TCL: (silica gel, 90% chloroform/10% methanol). RF (IV): 0.78 and 0.70. RF (V): 0.28.

Step 5. Deacetylation of 5-(N-2,3-diacetoxypropyl-acetamido)-2,4,6-triiodo-N-(2-acetoxyethyl)-N'-(2,3-dihydroxypropyl)-isophthalamide (VII) into:
5-(N-2,3-dihydroxypropylacetamido)-2,4,6-triiodo-N-(2-hydroxyethyl)-N'-(2,3-dihydroxypropyl)-isophthalamide (VIII)

The product from the previous amidation reaction (VII) (220g) was dissolved in methanol (450ml) and 1M sodium methoxide in methanol (50ml) was added. The solution was stirred for 30min, during which time methyl acetate was removed as an azeotrope with methanol. The final solution was neutralized to pH 7.0 by the addition of Dowex-50-H+. The solution was freed or solvent to obtain (VIII) as an off-white foam (184g, 0.232 moles) (yield: 84% from the corresponding acid chloride). An aqueous solution of (VIII) (0.5 moles) was charcoaled (5% W/W) at 80°C for 4hrs, filtered, water removed, and the product recrystallized from 5% aqueous ethanol, to yield 87% of (VIII) 99.2% pure. (Analytical data: See Example 7).

Example 8. Alkylation of sodium iothalamate
5-Acetamido-2,4,6-triiodo-N-methylisophthalamic acid (XI) into:
5-(N-2,3-Dihydroxypropylacetamido)-2,4,6-triiodo-N-methylisophthalamic acid (XII)

Sodium iothalamate (XI) (146g, 229.5mMoles) was dissolved in 1N sodiuim hydroxide (380ml), followed by addition (over 30min) of 3-chloro-1,2-propanediol (28.75ml; 344mMoles); pH was adjusted with 5N NaOH to 11.5-12.0. The mixture was brought to 85°C and stirred for 2hr to completion by TLC. The pH was adjusted to 6-7 with concentrated hydrochloric acid and the water removed on an evaporator. The residue was azeotroped with toluene (100ml x 1) to give 215g (including inorganic salts) of an off-white product (XII) which without isolation was acetylated in the next reaction.

Example 9. Acetylation of the alkylated iothalamic acid
5-(N-2,3-Dihydroxypropylacetamido)-2,4,6-triiodo-N-methylisophthalamic acid chloride (XII) into:
5-(N-2,3-Diacetoxypropylacetamido)2,4,6-triiodo-N-methylisophthalamic acid (XIII)

To the crude product (XII) (215g) from Example 10 were added pyridine (25ml) followed by acetic anhydride (400ml) with the temperature maintained below 50°C. The mixture was heated at 50°C for 1hr and the solvents were removed in vacuo. The residue was co-evaporated with toluene (2 x 100ml) and dissolved in a mixture of ethyl acetate (300ml) and aqueous sodium bicarbonate (750ml). The aqueous layer was extracted with ethyl acetate (2 x 200ml) and acidified with concentrated hydrochloric acid to pH 0.5. The mixture was extracted with ethyl acetate (3 x 300ml) and the combined organic layers were washed with water (2 x 100ml) and brine (2 x 50ml) and dried (MgSO₄). Removal of the solvent gave the product (XIII), a light yellow foam (163g) (92% yield from sodium iothalamate (XI)).

Example 10. Acylchlorination of the alkylated, acetylated iothalamic acid
5-(N-2,3-Diacetoxypropylacetamido)-2,4,6-triiodo-N-methylisophthalamic acid (XIII) into:
5-(N-2,3-Diacetoxypropylacetamido)-2,4,6-triiodo-N-methylisophthalamic acid chloride (XIV)

The product (XIII) of Example 11 (163g, 0.21 mole) was dissolved in thionyl chloride (500ml), stirred and refluxed for 1hr, when TLC showed that the reaction was over. Thionyl chloride was distilled off at 50-60°C at 100 Torr and the residue dried by co-evaporation with ethyl acetate (2 x 100ml). The off-white foamy product was dissolved in ethyl acetate (700ml), washed with saturated aqueous sodium bicarbonate (4 x 200ml) and brine (2 x 250ml). The organic layer was dried (MgSO₄) and the solvent removed to give the product (XIV) as an off-white foam (143.3g) representing 79% yield as calculated from the iothalamic acid.

Example 11. Amidation of alkylated, acetylated iothalamic acid chloride with protected D,L-aminothreitol
5-(N-2,3-Diacetoxypropylacetamido)-2,4,6-triiodo-N-methylisophthalamic acid chloride (XIV) into:
5-(N-2,3-Diacetoxypropylacetamido)-2,4,6-triiodo-N-(trans-2,2-dimethyl-6-hydroxyl-1,3-dioxepan-5-yl)-N'-methylisophthalamide (XVII)

To the solution of the title compound (XIV) (11g, 13.9mMoles) in dimethylacetamide (25ml) were added trimethylamine (1.9ml; 13.9mMoles) and trans-5-amino-2,2-dimethyl-6-hydroxy-1,3-dioxepane (2.69g, 16.7mMoles). The reaction mixture was stirred at room temperature for 8hr to completion by TLC. The solvent was removed in vacuo and the residue dissolved in ethyl acetate (50ml). The solution was washed with water (3 x 25ml) and brine (2 x 25ml). Drying (MgsO₄), followed by solvent removal, gave the product (XVII) as a pale yellow foam.

Example 12 . Deprotection of alkylated, acetylated iothalamaic acid amidated with trans-dioxepane to D,L-aminothreitol derivative
5-(N-2,3-Diacetoxypropylacetamido)-2,4,6-triiodo-N-(trans-2,2-dimethyl-6-hydroxy-1,3-dioxepan-5-yl)-N'-methylisophthalamide (XVII) into:
5-(N-2,3-Dihydroxypropylacetamido)-2,4,6-triiodo-N-methyl-N'-(1,3,4-trihydroxy-threo-but-2-yl)-isophthalamide (XVIII)

To a solution of the title compound (XVII) (4.5g, 4.92mMoles) in methanol (15ml) was added 5N NaOH to pH 13. By TLC, deacetylation was complete after 30min at 24°C. The solution was treated with Dowex 50-H⁺ resin and the solvent removed on a rotary evaporator to give 4.30g foam, which was dissolved in H₂O (30ml). 1N HCl (30ml) was added and the solution stirred for 1hr at 25°C. The solvents were removed on a rotary evaporator, and the residual acid removed with Dowex mixed-bed resin (AG-501). Charcoaling and evaporation gave the product (XVIII) as a white foam (3.6g) (93% yield).

Example 13. Amidation of alkylated, acetylated ioxithalamic acid chloride with 3-amino-1,2-propanediol
5-(N-2,3-Diacetoxypropylacetamido)-2,4,6-triiodo-N-(2-acetoxyethyl)-isophthalamic acid chloride (IV) into:
5-(N-2,3-Diacetoxypropylacetamido)-2,4,6-triiodo-N-(2,3-dihydroxyproypl)-N'-(2-acetoxyethyl)-isophthalamide (VII)
(Refer also to Example 7, Alternate Synthesis)

The title compound (IV) (240g, 0.27mMoles) in 1,2-dichloroethane (total volume 160ml) was diluted with acetone (270ml) and added to a mixture of 3-amino-1,2-propanediol (30.4g, 0.334mMoles), water (65ml), and sodium bicarbonate (23.4g, 0.278mMoles). The mixture was heated at 55°C for 8hrs, when TLC indicated the reaction was complete. Water (500ml) was added and the solution was extracted with 1,2-dichlorethane (2 x 40ml) containing 15% (by volume) of acetone. The aqueous layer was salted with sodium sulfate (140g) and extracted with a mixture of dichloromethane: n-propanol (9:1, 300ml). The dichloromethane was distilled off, n-propanol (300ml) was added, the solution concentrated to 250ml, and treated with Dowex-50-H+ resin, and charcoaled for 6hrs under reflux. Filtration and solvent removal gave (VII) (220g, 86% yield).

Example 14. Deprotection of alkylated, acetylated ioxithalamic acid amidated with 3-amino-1,2-propanediol
5-(N-2,3-Diacetoxypropylacetamido)-2,4,6-triiodo-N-(2,3-dihydroxypropyl)-N'-(2-acetoxyethyl)-isophthalamide (VII) into:
5-(N-2,3-Dihydroxypropylacetamido)-2,4,6-triiodo-N-(2,3-dihydroxypropyl)-N'-(2-hydroxyethyl)-isophthalamide (VIII)
(Refer also to Example 7, Alternate Synthesis)

The product of the previous amidation reaction (VII) (220g) was dissolved in methanol (450ml) and 1M sodium methoxide in methanol (50ml) was added. The solution was stirred for 30min, while methyl acetate was continuously removed in vacuo, then neutralized to pH 7.0 by Dowex-50-H+. Solvent removal gave a solid (VIII) (184g, 0.232 moles) (yield: 84% from (IV) acid chloride).

Example 15. Acetylation of 5-amino-2,4,6-triiodo-N-(2,3-dihydroxypropyl)-isophthalamic acid
5-amino-2,4,6-triiodo-N-(2,3-dihydroxypropyl)-isophthalamic acid (XXX) into:
5-acetamido-2,4,6-triiodo-N-(2,3-diacetoxypropyl)-isophthalamic acid (XXXI)

The title compound (XXX) (252.8g, 0.4 moles) was mixed with glacial acetic acid (150ml) and acetic anhydride (350ml, 3.7 moles). Concentrated sulfuric acid was added (10ml) and the solution was heated for 6hr at 40°C. The suspension was poured into a mixture of ice and brine (2:1, 1.5L) stirred for 30min and filtered off. The solid was washed with cold water (200ml x 1) and dried to give 274g (90% yield of (XXXI).

Example 16. Alkylation, followed by acetylation of 5-acetamido-2,4,6-triiodo-N-(2,3-dihydroxypropyl)-isophthalamic acid
5-acetamido-2,4,6-triiodo-N-(2,3-diacetoxypropyl)-isophthalamic acid (XXXI) into:
5-(N-2,3-diacetoxypropylacetamido)-2,4,6-triiodo-N-(2,3-diacetoxypropyl)-isophthalamic acid (XXXII)

The title compound (XXXI) (227.4g, 0.3 moles) was dissolved in 1N sodium hydroxide (300ml) and pH adjusted to 12.0 by addition of 5N sodium hydroxide.
Calcium hydroxide (97%, 17g, 0.223 moles) and 3-chloro-1,2-propanediol (44.76g, 0.405 moles) were added at 85-90°C over two hours. After 2.5hrs the reaction was complete by TLC.
The pH was brought to 6.0 by concentrated hydrochloric acid (4ml), and water removed to give an oil which was dissolved in glacial acetic acid (500ml). The solution was concentrated by 50% and pyridine (24.2ml, 0.3 moles) and acetic anhydride (311ml, 3.3 moles) were added over 45min. After 6hr at 70°C, TLC indicated the reaction was complete.
Upon volume reduction to 50%. ice-cold water (500ml) and ethyl acetate (250ml) were added, the layers separated and the aqueous layer acidified to pH 1.0 with concentrated hydrochloric acid (60ml). The product was extracted into dichloromethane (500ml), which was then replaced with 1,2-dichloroethane (400m1) to give 211.6g, 0.231 moles of (XXXII) (77% yield).

Example 17. Chlorination of alkylated, acetylated 5-acetamido-2,4,6-triiodo-N-(2,3-dihydroxyproyl)-isophthalamide
5-N-(2,3-diacetoxypropylacetamido)-2,4,6-triiodo-N-(2,3-diacetoxypropyl)-isophthalamic acid (XXXII) into:
5-N-(2,3-diacetoxypropylacetamido)-2,4,6-triiodo-N-(2,3-diacetoxypropyl)-isophthalamic acid chloride (XXXIII)

To a solution of the title compound (XXXII) (169.5g, 0.185 moles) in 1,2-dichloroethane (total volume 450m1) at 55°C was added thionyl chloride (51.25ml, 0.702 moles). The solution was heated at 70°C for 3hr, when TLC showed the reaction was complete.

The solution was concentrated to 250ml and the residue azeotroped with 1,2-dichloroethane (200ml x 2). 700ml of 1,2-dichloroethane was added and the mixture washed with saturated sodium bicarbonate (500ml x 1) to give (XXXIII) (165.25g, 96% yield).

Example 18. Amidation of alkylated, acetylated 5-acetamido-2,4,6-triiodo-N-(2,3-dihydorxypropyl)-isophthalamic acid chloride with 2-aminoethanol 5-(N-2,3-diacetoxypropylacetamido)-2,4,5-triiodo-N-(2,3-diacetoxypropyl)-isophthalamic acid chloride (XXXIII) into:

5-(N-2,3-diacetoxypropylacetamido)-2,4,6-triiodo-N-(2-hydroxyethyl)-N'-(2,3-diacetoxypropyl)-isophthalamide (XXXIV)

The title' compound (XXXIII), (132g, 0.141Moles) was dissolved in acetone (300ml) and water (75ml), and to this was added sodium bicarbonate (11.85g, 0.141Moles) and 2-aminoethanol (10.35g, 0.170Moles). The reaction mixture was heated at 50°C for 6hr, when TLC showed that the reaction was complete.

The reaction mixture was diluted with water (500ml) and toluene (200ml), and the layers were separated. The organic layer was back-extracted with water (100mlx1), the aqueous extracts combined and satuarated with sodium chloride, and the product was extracted with dichloromethane (400ml). The dichloromethane layer was washed with 50% brine solution (50mlx1), the layers separated and the dichloromethane removed to give XXXIV (120.6g, 89% yield).

Example 19. Deprotection of alkylated, acetylated 5-(N-2,3-dihydroxypropylacetamido)-2,4,6-triiodo-isopthalamic acid amidated with 2-amino ethanol 5-(N-2,3-diacetoxypropylacetamido)-2,4,6-triiodo-N-(2-hydroxyethyl)-N'-(2,3-diacetoxypropyl)-isophthalamide (XXXIV) into:

5-(N-2,3-dihydroxypropylacetamido)-2,4,6-triiodo-N-(2-hydroxyethyl)-N'-(2,3-dihydroxypropyl)-isophthalamide (VIII)

The title compound (XXXIV) (60.3g, 0.063 moles) was dissolved in methanol (total volume 250ml). 15ml of a 1M solution of sodiuim methoxide were added and the solution stirred for 30min at 25°C while the methyl acetate generated was continuously distilled off in vacuo. The solution was then neutralized with Dowex 50-H+ and the solvent removed to give VIII (49.1g, 99% yield).

Example 20. Alkylation of acetylated ioxithalamic acid 5-acetamido-2,4,6-triiodo-N-(2-acetoxyethyl)-isophthalamic acid (XXXV) into:

5-(N-2,3-dihydroxypropylacetamido)-2,4,6-triiodo-N-(2-hydroxyethyl)-isophthalic acid (II)

The title compound (XXXV) (6.86g, 0.01 moles) was dissolved in 1N sodium hydroxide (10ml) and 10N sodium hydroxide (1ml) was added to saponify the ester.

The solution was heated to 90°C and calcium hydroxide (97%, 0.556g, 0.0075 moles) was added followed by 3-chloro-1,2-propanediol (1.5g, 0.0135 moles) over 1 hour. The reaction was heated for an additional 30 minutes to completion by TLC.

Glacial acetic acid was added to pH 5.0, solvents were evaporated and the residue azeotroped with toluene (20ml) to obtain 11.7g of a mixture amenable to acetylation as shown in Example 2 or step 2 or the alternative Synthesis of Example 7.

ALTERNATIVE SYNTHESIS OF COMPOUND VIII: EXAMPLES 21-23

Example 21. Iodination of 5-amino-N-(2-hydroxyethyl)-N'-(2,3-dihydroxypropyl)-isophthalamide (hydrochloride) 5-amino-N-(2-hydroxyethyl)-N'-(2,3-dihydroxypropyl)-isophthalamide (hydrochloride) (XXXVI) into:

5-amino-2,4,6-triiodo-N-(2-hydroxyethyl)-N'-(2,3-dihydroxypropyl)-isophthalamide (XXXVII):

The title compound (XXXVI, 600mg, 1.80mMoles was dissolved in water (8.9ml) and conc. hydrochloric acid (0.15ml). 1.84M KICl$_2$ (3.3ml) was added and the reaction was heated at 80°C For 3 hours. Reaction pH was adjusted with sodium bicarbonate, rotovaped to dryness, and dissolved in 8ml ethanol. Inorganic salts were filtered off, the filtrate acidified with conc. HCl, and evaporated to give 918mg of an orange solid (79% yield).

Example 22: Acetylation of 5-amino-2,4,6-triiodo-N-(2-hydroxyethyl)-N'-(2,3-dihydroxypropyl)-isophthalamide 5-amino-2,4,6-triiodo-N-(2-hydroxyethyl)-N'-(2,3-dihydroxypropyl)-isophthalamide (XXXVII) into:

5-acetamido-2,4,6-triiodo-N-(2-acetoxyethyl)-N'-(2,3-diacetoxypropyl)-isophthalamide (XXXVIII):

The title compound (600mg, 0.89mMoles) was dissolved into glacial acetic acid (1ml); pyridine (72$\mu$L, 0.89mMoles) and acetyl chloride (620$\mu$L, 8.9mMoles) were added and the reaction was heated at 50°C for 2 hours to completion by TLC.

The excess acetyl chloride was removed by distillation, the product dissolved in tetrahydrofuran (10ml) and the solution was washed with a brine-0.1 N HCl mixture (5mlx1). The THF was removed to give 650mg of the product (XXXVIII) (87% yield).

Example 23: Alkylation of 5-acetamido-2,4,6-triiodo-N-(2-acetoxyethyl)-N'-(2,3-diacetoxypropyl)-isophthalamide with epichlorohydrin

5-acetamido-2,4,6-triiodo-N-(2-acetoxyethyl)-N'-(2,3-diacetoxypropyl)-isophthalamide (XXXVIII) into:

5-N-(2,3-dihydroxypropyl)-acetamido-2,4,6-triiodo-N-(2-hydroxyethyl)-N'-(2,3-dihydroxypropyl)-isophthalamide (VIII)

The title compound (XXXVIII, 650mg, 0.77mMoles) was dissolved in 1,2-propanediol (3ml), sodium bicarbonate (1.29mg, 1.54mMoles) and epichlorohydrin (1.2ml, 15.4mMoles) were added and the reaction was heated at 90°C. TLC and HPLC showed that the reaction was complete at 1 hour to yield compound VIII, in 73% yield.

The above procedures demonstrated the simple, rapid and efficient synthesis strategy of the subject invention. The economics of the method are evidenced by high yields and use of intermediates without further purification. In addition, only simple inexpensive and readily removable reagents are employed and the resulting product is substantially free of impurities. The number of steps from the starting material is minimal to further minimize separations and purifications.

All compounds were tested for stability, solubility, osmolality, viscosity and systemic toxicity, using conventional tests. Compound VIII was tested with existing compounds serving as control and shown to have substantially reduced osmolality while having comparable or superior properties in the other categories.

TABLE: Properties of Preferred Novel Compounds and of the Prior Art Non-Ionic CM*

| | Compound VIII | Iopromide | Iohexol | Iopamidol |
|---|---|---|---|---|
| Osmolality (mOsm/kg) | 524 | 607[+] | 690[+] | 619[+] |
| Viscosity (cps) | 4.9 | 4.8[+] | 6.1[+] | 4.5[+] |
| i.v. LD50(gI/kg)[o] Mice (female CD-1) | 18.8 (18-19.5)[++] | 11.5-13.0 | 17.9 (17.2-18.6)[++] | 17-18.5 |
| Rats (female Lewis) | 14-16.5 | 10-11.5 | 13.5-15 | 12.2-13 |

\* All at 300mg I/ml concentration and 37°C. Injection rates 1ml/min in mice and 5ml/min in rats.

+ Ref. Handbook of Experimental Pharmacology, Vol. 73, M. Sovak, ed., Springer-Verlag 1984, Table 1, p. 9.

o Ref. Salvesen, S. in Acta Radiol. Suppl. 362, p. 73, 1980.

++ Confidence limit, indicating no satistically significant difference.

It is evident from the above results that the subject compounds provide improvement in contrast media since, in angiography, hyperosmolality causes vascular pain and contrast media solutions of less than 600mOsm are known to be painless. The combination of low viscosity with low osmolality has never previously been obtained in a clinically useful contrast medium.

All publications cited in this specification are indicative of the level of skill of those skilled in the art to which this invention pertains.

## Claims

1. 5-(N-2,3-Dihydroxypropylacetamido)-2,4,6-triiodo-N-(2,3-dihydroxypropyl)-N'-(2-hydroxyethyl)-isophthalamide.

2. 5-(N-acetamido)-2,4,6-triiodo-N-(2,3-dihydroxypropyl)-N'-(2-hydroxyethyl) isophthalamide.

3. 5-(N-2,3-Dihydroxypropylacetamido)-2,4,6-triiodo-N-methyl-N'-(1,3,4-trihydroxy-threo-but-2-yl)-isophthalamide.

4. Use of a compound of claim 1 or claim 3 for the manufacture of an X-ray contrast medium.

5. A contrast medium formulation comprising 5-(N-2,3-dihydroxypropylacetamido)-2,4,6-triiodo-N-(2-3,-dihydroxypropyl)-N'-(2-hydroxyethyl)-isophthalamide in a physiologically acceptable medium at a concentration in the range of about 50 to 400 mg I/ml.

6. A contrast medium formulation comprising 5-(N-2,3-dihydroxypropylacetamido)-2,4,6-triiodo-N-methyl-N'-(1,3,4-trihydroxy-threo-but-2-yl)-isophthalamide in a physiologically acceptable medium at a concentration in the range of about 50 to 400 mg I/ml.

**Patentansprüche**

1. 5-(N-2,3-Dihydroxypropylacetamido)-2,4,6-trijod-N-(2,3-dihydoxypropyl)-N'-(2-hydroxyäthyl )isophthalamid.

2. 5-(N-Acetamido)-2,4,6-trijod-N-(2,3-dihydroxypropyl)-N'-(2-hydroxyäthyl )isophthalamid.

3. 5-(N-2,3-Dihydroxypropylacetamido)-2,4,6-trijod-N-methyl-N'-(1,3,4-trihydroxy-threo-but-2-yl )isophthalamid.

4. Verwendung einer Verbindung nach Anspruch 1 oder 3 zur Herstellung eines Röntgenstrahlen-Kontrastmediums.

5. Kontrastmediumformulierung, die 5-(N-2,3-Dihydroxypropylacetamido)-,2,4,6-trijod-N-(2-3-dihydroxypropyl)-N'-(2-hydroxyäthyl)-isophthalamid in einem physiologisch verträglichen Medium in einer Konzentration im Bereich von etwa 50 bis 400 mg Jod/ml enthält.

6. Kontrastmediumformulierung, die 5-(N-2,3-Dihydroxypropylacetamido)-2,4,6-trijod-N-methyl-N'-(1,3,4-trihydroxythreo-but-2-yl)isophthalamid in einem physiologisch verträglichen Medium in einer Konzentration im Bereich von etwa 50 bis 400 mg Jod/ml enthält.

**Revendications**

1. 5-(N-2,3-dihydroxypropylacétamido)-2,4,6-triiodo-N-(2,3-dihydroxypropyl)-N'-(2-hydroxyéthyl)-isophtalamide.

2. 5-(N-acétamido)-2,4,6-triiodo-N-(2,3-dihydroxypropyl)-N'-(2-hydroxyéthyl)isophtalamide.

3. 5-(N-2,3-dihydroxypropylacétamido)-2,4,6-triiodo-N-méthyl-N'-(1,3,4-trihydroxy-thréo-but-2-yl)-isophtalamide.

4. Utilisation d'un composé de la revendication 1 ou de la revendication 3, pour la fabrication d'un milieu de contraste pour rayons X.

5. Formule d'un milieu de contraste comprenant du 5-(N-2,3-dihydroxypropylacétamido)-2,4,6-triiodo-N-(2,3-dihydroxypropyl)-N'-(2-hydroxyéthyl) isophtalamide, dans un milieu physiologiquement acceptable ,à une concentration comprise entre environ 50 et 400 mg I/ml.

6. Formule d'un milieu de contraste comprenant du 5-(N-2,3-dihydroxypropylacétamido)-2,4,6-triiodo-N-méthyl-N'-(1,3,4-trihydroxy-thréo-but-2-yl)isophtalamide, dans un milieu physiologiquement acceptable,à une concentration comprise entre environ 50 et 400 mg I/ml.